# EUROPÄISCHE PATENTSCHRIFT

(11) **EP 0 345 498 B1**
(45) Veröffentlichungstag und Bekanntmachung des Hinweises auf die Patenterteilung: **15.03.1995**
(21) Anmeldenummer: 89108929.4
(22) Anmeldetag: 18.05.1989
(51) Int. Cl.: H01T 23/00, A61N 1/10

(54) **Gerät zur Entkeimung und Desodorisierung von Räumen**
Sterilisation and deodorization device for spaces
Dispositif de stérilisation et de désodorisation d'espaces

(30) Priorität: 07.06.1988 CH 2163/88
(43) Veröffentlichungstag der Anmeldung: 13.12.1989
(73) Patentinhaber: Zellweger, Max, CH-8805 Richterswil (CH)
(72) Erfinder: Zellweger, Max, CH-8805 Richterswil (CH)
(74) Vertreter: Althoff, Gerhard

(56) Entgegenhaltungen:
- EP-A- 0 053 647
- DE-A- 2 064 690
- DE-A- 2 413 275
- DE-A- 3 106 187

## Beschreibung

Die Erfindung bezieht sich auf ein Gerät zur Entkeimung und Desodorisierung von Räumen, bestehend aus einem mit einem Transformator versehenen elektrischen Schaltungsteil zur Erzeugung von Hochspannung und Hochfrequenz und einem mit einer ersten Elektrode sowie einer elektrisch isoliert dazu angeordneten zweiten Elektrode versehenen Ionisationsteil, an welches zur Entladung einer Koronaentladung die Hochspannung angelegt ist.

Geräte zur Entkeimung von Räumen sind verschiedenen Ausführungen bekannt. Trotz ihres oft stark verschiedenen Aufbaus erzeugen diese Geräte vor allem Ozon (O₃). Ozon hat eine indirekte, keimtötende Wirkung, jedoch ist Ozon eines der stärksten Oxidationsmittel, welches die Schleimhäute des menschlichen Organismus, insbesondere der Atemwege, angreift und zu lästigen Reizungen führt.

Für die Erzeugung dieser Luftionisation werden Entladungsröhren verwendet, bei denen an einem Isolator, meistens an einer Glasröhre, an der Innen- und an der Aussenwand je eine Elektrode angelegt wird. An diese beiden gegenüberliegenden Elektroden wird Hochspannung angelegt, die zu Entladungskoronaren mit einer entsprechenden Elektronenemission führen.

Aus der CH-A 666 372 ist ein Verfahren und eine Vorrichtung zum Erzeugen von Koronaentladungen in Luft bekannt, bei welche eine Isolatorröhre verwendet wird, in deren Innenraum in der Nähe der Innenwand eine hülsenförmige erste Elektrode angeordnet ist. Eine zweite hülsenförmige Elektrode wird auf die Aussenwand der Isolatorröhre geschoben, welche aus einem Drahtgitter geformt ist und an welcher nach Anlegen einer Hochspannung Koronaren entstehen, welche eine Luftionisation zur Folge hat. Mit diesem Gerät erfolgt die Luftionisation in Form von Sauerstoff-Cluster, deren Verträglichkeit grösser ist als von Ozon (O₃).

Da jedoch die Isolatorröhre und er dazu gehörende Transformator mit seiner Steuerung ein verhältnismässig grosses Volumen aufweist, sind Geräte dieser Art in kleineren Räumen, wie sie etwa bei Kühlschränken vorliegen, nicht anwendbar, im übrigen auch deshalb nicht, weil bei der benötigten Hochspannung ein amtlich vorgeschriebener Berührungsschutz verlangt wird. Die bekannte Vorrichtung ist deshalb in einem Gehäuse angeordnet und dient der Luftdesinfektion, der Desodorisierung und er Aufbereitung von verbrauchter Luft in grösseren Räumen.

Aus der EP-A 0 053 647 ist zur Erzeugung eines bioklimatisch wirksamen elektrischen Feldes ein als geschlossenes Gehäuse ausgebildetes Gerät bekannt, bei welchem in dem Gehäuse im wesentlichen ein Generator, eine Batterie sowie eine Elektrode angeordnet sind, wobei die Elektrode aus einer leitfähigen Beschichtung besteht und an der Innenfläche des aus nichtleitfähigem Material gebildeten Gehäusedeckels angeordnet ist.

Weiterhin ist aus der DE-A 3 106 187 ein sogenannter Dünnschicht-Ionisator zum Ionisieren von Luft bekannt, welcher im wesentlichen zwei in parallelem Abstand zueinander angeordnete Isolatorstreifen aufweist, bei welchen an der Oberfläche des einen Isolatorstreifens im Abstand zueinander angeordnete und als Emitterstruktur mit Zahnelementen ausgebildete Verbinderabschnitte befestigt sind. Die gesamte, mit Luftspalten versehene Struktur ist bei diesem Ionisator in Form streifenförmiger und verhältnismässig flexibler Bänder ausgebildet.

Der Erfindung liegt die Aufgabe zugrunde, ein Gerät gemäss der im Oberbegriff des Patentanspruchs genannten Art zur Desinfektion, Desodorisierung und Aufbereitung der Luft in Räumen durch Elektronenemission dahingehend auszubilden, dass es als kleines, kompaktes Gerät mit einem einwandfreien Berührungsschutz und zudem gegen Temperatur und Feuchtigkeitseinflüsse geschützt ist.

Diese Aufgabe wird dadurch gelöst, dass das elektrische Schaltungsteil auf der einen Seite einer Leiterplatte und unmittelbar auf der anderen Seite derselben die als ebene Platte ausgebildete erste Elektrode sowie eine Isolierplatte angeordnet sind, wobei die Isolierplatte und die erste Elektrode sowie die damit in Verbindung stehende Leiterplatte zusammen mit dem daran angeordneten elektrischen Schaltungsteil in dieser Reihenfolge sowie in Schichtbauweise in der aus einem geerdeten Metallsieb oder Metallgitter bestehenden und als Haube ausgebildeten zweiten Elektrode angeordnet sind.

Im Betrieb des erfindungsgemässen Gerätes wird an den Maschen der als Metallsieb oder Metallgitter ausgebildeten zweiten Elektrode eine Elektronen-Emittierung erreicht, dass dabei in Verbindung mit Sauerstoff-Atomen negative Sauerstoff-Ionen gebildet werden, durch welche die in der Umgebungsluft befindlichen Bakterien und Sporen abgetötet und Gerüche neutralisiert werden. Das Gerät arbeitet für das menschliche Gehör geräuschlos und verursacht zudem keine auf die Schleimhäute und Atemwege des menschlichen Organismus störend wirkende Reizungen oder dergleichen. Das Gerät kann in Räumen, in welchen Verhältnisse bezüglich Temperatur und Feuchtigkeit vorliegen, eingesetzt und funktionssicher betrieben werden.

Weitere Merkmale der Erfindung ergeben sich aus der folgenden Beschreibung in Verbindung mit der Zeichnung. Die Erfindung wird nachstehend in Verbindung mit der Zeichnung beschrieben. Es zeigt:
- Fig.1: ein in perspektivischer Ansicht und als Sprengzeichnung dargestelltes Gerät zur Entkeimung und Desodorisierung von Räumen,
- Fig.2: eine als Blockschaltbild dargestellte elektrische Schaltung für das Gerät gemäss Fig.1, und
- Fig.3: das in perspektivischer Ansicht und in zusammengebautem Zustand dargestellte Gerät gemäss Fig.1.

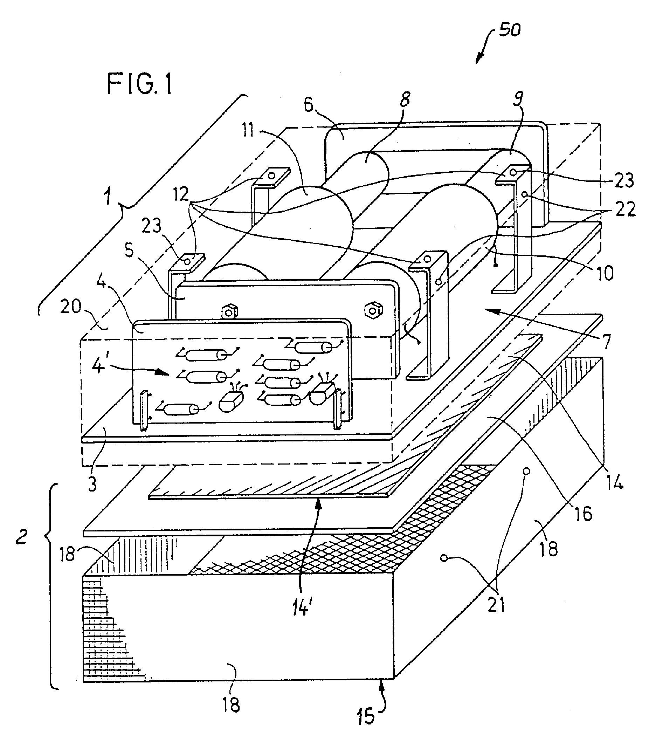

Fig. 1 zeigt ein in perspektivischer Ansicht und als Sprengzeichnung dargestelltes und in der Gesamtheit mit 50 bezeichnetes Gerät zur Entkeimung und Desodorisierung von Räumen, welches ein in der Gesamtheit mit 1 bezeichnetes Schaltungsteil und ein in der Gesamtheit mit 2 bezeichnetes Ionisationsteil umfasst. In zusammengesetztem Zustand der beiden Teile 1 und 2 bilden diese einen quaderförmigen Körper von weniger als 10 cm Länge und 10 cm Breite sowie eine Höhe von weniger als 4 cm.

Aus Fig. 1 sind die einzelnen, schichtweise übereinanderliegenden Elemente des Schaltungsteils 1 zu erkennen. Auf einer ersten Leiterplatte 3 (Grund-Print) ist eine zweite Leiterplatte 4 (Elektronik-Print) angeordnet, welche sämtliche elektronischen Komponenten 4′ enthält, die für den Betrieb des Gerätes 50 erforderlich sind. Die zweite Leiterplatte 4 kann auch in die erste Leiterplatte 3 integriert sein, so dass dann nur eine Leiterplatte benötigt wird.

An zwei im Abstand zueinander angeordneten Halterplatten 5, 6 ist ein Transformator 7 befestigt, an dessen beiden Schenkeln 8 und 9 Wicklungen 10 und 11 angeordnet sind, wobei die eine Primärwicklung 10 auf dem einen Schenkel 9 und die andere Sekundärwicklung 11 auf dem anderen Schenkel 8 angeordnet ist.

Auf der ersten Leiterplatte 3 sind im Abstand zueinander angeordnete Montagebügel 12 abgestützt, mit Hilfe welcher das Entkeimungsgerät 50 auf einer nicht dargestellten Unterlage befestigt werden kann. Die einzelnen Montagebügel 12 sind auf zwei Seiten der ersten Leiterplatte 3 angeordnet, jedoch könnten diese auch in anderer Weise angeordnet sein. Zweckmässig liegen sie innerhalb der Fläche der ersten Leiterplatte 3. Die Montagebügel 12 können auch als Teile einer seitlichen Wand (nicht dargestellt) ausgebildet sein.

Auf der dem Transformator 7 entgegengesetzten Seite der ersten Leiterplatte 3 ist eine erste Elektrode 14 des Ionisationsteils 2 angeordnet. Die erste Elektrode 14 ist eine massive oder gelochte ebene Platte aus einem Metall, vorzugsweise der Gruppe Stahl, Kupfer, Aluminium und Messing. Die erste Elektrode 14 kann aber auch als Film auf die Oberfläche einer Vergussmasse 20 aufgedampft werden, derart, dass die erste Elektrode 14 eine freie Kontaktierungsfläche 14′ aufweist.

Der Kontaktierungsfläche 14′ der ersten Elektrode 14 ist eine Isolierplatte 16 zugeordnet, die aus einem Isolationsmaterial, beispielsweise Glas oder Kunststoff hergestellt ist. Wie aus Fig. 1 ersichtlich ist, weist diese Isolierplatte 16 etwas grössere Dimensionen als die erste Elektrode 14 auf und überragt somit die erste Elektrode 14 auf ihrem gesamten Umfang.

Während die erste Elektrode 14 aus einem verhältnismässig dünnen Blech oder Film hergestellt ist, weist die Isolierplatte 16 eine grössere Dicke auf, beispielsweise 0,5 - 4,0 mm auf.

Auf die Isolierplatte 16 ist eine zweite Elektrode 15 aufgesetzt. Diese ist aus einem Metall, vorzugsweise der Gruppe Stahl, Kupfer, Aluminium oder Messing hergestellt. Die zweite Elektrode 15 ist aus Drähten der genannten Metalle zusammengesetzt und bildet ein Sieb oder Gitter, an dessen Maschen beim Betrieb des Gerätes 50 Elektronen emittiert werden und bei der Verbindung mit Sauerstoff-Atomen negative Sauerstoff-Ionen bilden.

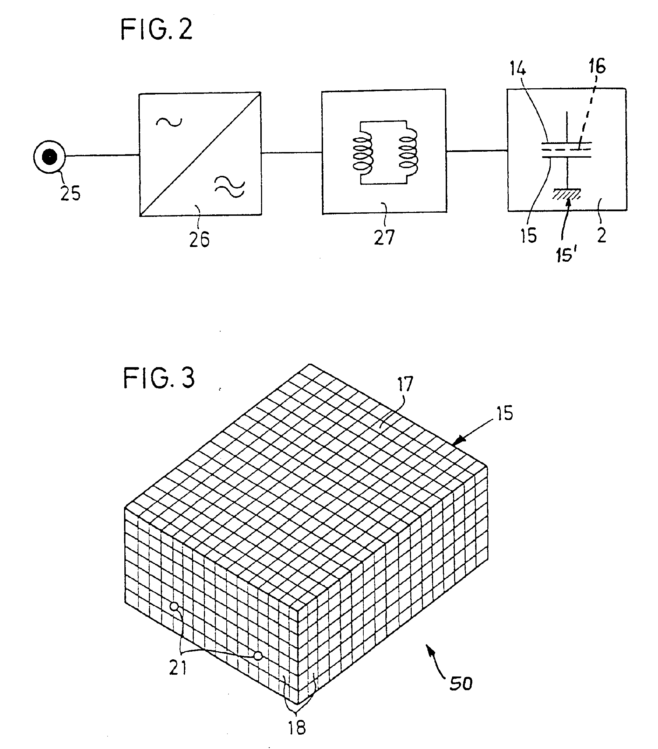

Die zweite Elektrode 15 setzt sich aus einer ebenen Grundfläche 17 und Seitenflächen 18 zusammen und bildet somit eine Haube, durch welche die Isolierplatte 16, die erste Elektrode 14 und das Schaltungsteil 1 abgedeckt sind (Fig. 3). Damit die zweite Elektrode 15 nicht durch eine Flüssigkeit oder ein anderes Material verschmutzt wird, kann die zweite Elektrode 15 durch einen nicht dargestellten Schutzkorb abgedeckt werden, welcher Durchbrechungen aufweist, damit die Elektronenemission in die Luft nicht behindert wird.

Damit das Schaltungsteil 1 vollständig geschützt ist, wird dieses vollständig ausgegossen, wozu die erwähnte Ausgussmasse 20 verwendet wird. Diese ist ein thermoplastischer oder duroplastischer Kunststoff, der nach dem Vergiessen aushärtet. Die Ausgussmasse 20 erstreckt sich bis zu den Oberkanten der einzelnen Montagebügel 12 bzw. bis zu der nicht dargestellten Wand, die zwar teilweise in der Ausgussmasse eingebettet sind, jedoch soweit zugänglich sind, dass die zweite Elektrode 15 an den Montagebügeln 12 bzw. der Wand befestigt werden kann. Hierzu sind Bohrungen oder Gewindelöcher 21, 22 vorgesehen, mit Hilfe welcher die Befestigung der zweiten Elektrode 15 mit den Montagebügeln 12 bzw. der nicht dargestellten Wand erfolgen kann. Die Montagebügel 12 bzw. die Wand sind an ihrem freien Ende abgewinkelt und weisen dort je eine Bohrung 23 auf. Das zu einem Würfel vergossene Gerät 50 kann somit auf einer nicht dargestellten Unterlage befestigt werden, wobei die die Bohrungen 23 durchdringenden, nicht dargestellten Bolzen und damit auch die zweite Elektrode 15 geerdet sind, so dass damit die amtliche Vorschrift über den Berührungschutz erfüllt ist. Die erste Leiterplatte 3 kann auch beidseitig von der Ausgussmasse 20 umgeben sein.

In Fig. 2 ist die elektronische Schaltung als Blockschaltbild dargestellt. Von einer elektrischen Stromquelle 25, welche beispielsweise das öffentliche Netz von 220 Volt oder eine Gleichstromquelle sein kann, wird die Spannung mit einem Wandler 27 auf etwa drei Kilovolt (KV) transformiert. Mit einer Zerhackerschaltung 26 wird die für den Wandler 27 erforderliche Frequenz erzeugt. Diese Frequenz ist von der lonisationsfläche der zweiten Elektrode 15 des lonisationsteiles 2 abhängig und muss auf Resonanz abgestimmt werden. Der Wandler 27 kann hierbei ein Durchfluss-, Gegentakt- oder Resonanz-Wandler sein, dessen Sekundärkreis als Resonanzkreis ausgebildet ist und mit einem Anschluss mit der ersten Elektrode 14 und mit dem zweiten Anschluss mit der Schutzerde verbunden ist. Die benötigten Frequenzen betragen ca. 10 Khz. In dem in Fig. 2 schematisch dargestellten Ionisationsteil 2 ist die Erdung 15′ der zweiten Elektrode 15 sowie die zwischen den beiden Elektroden 14 und 15 angeordnete Isolierplatte 16 angedeutet.

In Fig. 3 ist die äussere Ausgestaltung des Entkeimungsgerätes 50 dargestellt und man erkennt die zweite Elektrode 15 mit ihrer Grundfläche 17 und den Seitenflächen 18 sowie die im Abstand zueinander angeordneten Gewindelöcher 21.

Das beschriebene Gerät 50 erfüllt die Voraussetzungen eines störungsfreien Betriebes in verschiedenen Räumen, z.B. in Kühl- und Gefrierräumen, aber auch in Autos, Telefonkabinen und WC-Anlagen. Bei einer Grösse des Gerätes 50 von 90×90×35 mm wird eine Sekundärleistung von zwei Watt und eine Spannung von drei KV erreicht. Die nutz bare Isonisationsfläche beträgt hierbei 49 cm². Die Ionisationsfläche ist im wesentlichen der aktive Teil der zweiten Elektrode 15. Die im Betrieb auftretende Elektronenemission erzeugt negative Ionen, insbesondere Sauerstoff-Ionen. Diese haben die Fähigkeit, Bakterien und Sporen in der Luft abzutöten sowie Gerüche zu neutralisieren und damit Geruchübertragungen zu verhindern, z.B. Zwiebel- oder Käsegeruch auf Milch oder Schlagrahm.

## Patentansprüche

1. Gerät zur Entkeimung und Desodorisierung von Räumen, bestehend aus einem mit einem Transformator (7) versehenen elektrischen Schaltungsteil (1) zur Erzeugung von Hochspannung und Hochfrequenz und einem mit einer ersten Elektrode (14) sowie einer elektrisch isoliert dazu angeordneten zweiten Elektrode (15) versehenen Ionisationsteil (2), an welches zur Entstehung einer Koronaentladung die Hochspannung angelegt ist, dadurch gekennzeichnet, dass das elektrische Schaltungsteil (1) auf der einen Seite einer Leiterplatte (3) und unmittelbar auf der anderen Seite derselben die als ebene Platte ausgebildete erste Elektrode (14) sowie eine Isolierplatte (16) angeordnet sind, wobei die Isolierplatte (16) und die erste Elektrode (14) sowie die damit in Verbindung stehende Leiterplatte (3) zusammen mit dem daran angeordneten elektrischen Schaltungsteil (1) in dieser Reihenfolge sowie in Schichtbauweise in der aus einem geerdeten Metallsieb oder Metallgitter bestehenden und als Haube ausgebildeten zweiten Elektrode (15) angeordnet sind.

2. Gerät nach Anspruch 1, dadurch gekennzeichnet, dass die aus einer Grundfläche (17) und daran angeordneten Seitenflächen (18) haubenförmig ausgebildete zweite Elektrode (15) derart ausgebildet ist, dass die Isolierplatte (16), die erste Elektrode (14) sowie die Leiterplatte (3) mit dem elektrischen Schaltungsteil (1) in eingesetztem Zustand von den Seitenflächen (18) derart überdeckt sind, dass das elektrische Schaltungsteil (1) auf der der Grundfläche (17) der zweiten Elektrode (15) gegenüberliegenden Seite durch eine Vergussmasse (20) in Form eines geeigneten Kunststoffes, wie Polyvinylchlorid (PVC) oder Polyurethan (PU), verschlossen ist.

3. Gerät nach den Ansprüchen 1 und 2, dadurch gekennzeichnet, dass das elektrische Schaltungsteil (1) mit der Leiterplatte (3) und der ersten Elektrode (14) derart in der Vergussmasse (20) eingebettet sind, dass die erste Elektrode (14) auf der der Isolierplatte (16) zugewandten Seite eine freie Kontaktierungsfläche (14′) aufweist.

4. Gerät nach Anspruch 3, dadurch gekennzeichnet, dass die erste Elektrode (14) mit der einen Fläche auf die Leiterplatte (3) aufgeklebt ist und auf der der Isolierplatte (16) zugewandten Seite die freie Kontaktierungsfläche (14′) aufweist.

5. Gerät nach Anspruch 3, dadurch gekennzeichnet, dass die erste Elektrode (14) als Film auf die Vergussmasse (20) aufgedampft ist und auf der der Isolierplatte (16) zugewandten Seite die freie Kontaktierungsfläche (14′) aufweist.

6. Gerät nach Anspruch 1, dadurch gekennzeichnet, dass mit dem Transformator (7) des elektrischen Schaltungsteils (1) die angelegte Netzspannung von 220V (AC) oder eine Gleichspannung von 24V (DC) auf einen zehn- oder mehrfachen Wert, beispielsweise auf drei Kilovolt (kV), transformiert wird.

7. Gerät nach Anspruch 1, dadurch gekennzeichnet, dass das elektrische Schaltungsteil (1) mit einer Stromquelle in Verbindung steht und eine Zerhackerschaltung (26) aufweist, mittels welcher die erforderliche Frequenz für einen den beiden Elektroden (14,15) vorgeschalteten Wandler (27) erzeugt wird.

## Claims

1. Device for the sterilisation and deodorisation of spaces, consisting of an electric circuit member (1) provided with a transformer (7) for producing high voltage and high frequency and an ionisation member (2) which is provided with a first electrode (14) and with a second electrode (15) electrically insulated therefrom, to which ionisation member (2) the high voltage is applied so as to form a corona discharge, characterised in that the electric circuit member (1) is arranged on one side of a printed circuit board (3) and the first electrode (14) designed as a plane plate as well as an insulating plate (16) are arranged directly on the other side thereof, the insulating plate (16) and the first electrode (14) as well as the printed circuit board (3) connected thereto being arranged together with the electric circuit member (1) arranged thereon in this sequence and in layers in the second electrode (15) consisting of an earthed metal screen or metal grid and designed as a hood.

2. Device according to Claim 1, characterised in that the second electrode (15) designed in the form of a hood from a top face (17) and lateral faces (18) arranged thereon is designed in such a way that the insulating plate (16), the first electrode (14) and the printed circuit board (3) with the electric circuit member (1) are covered by the lateral faces (18) in the used state in such a way that the electric circuit member (1) is sealed on the side remote from the top face (17) of the second electrode (15) by a pouring compound (20) in the form of a suitable plastic such as polyvinylchloride (PVC) or polyurethane (PU).

3. Device according to Claims 1 and 2, characterised in that the electric circuit member (1) with the printed circuit board (3) and the first electrode (14) is embedded in the pouring compound (20) in such a way that the first electrode (14) has a free contact face (14′) on the side facing the insulating plate (16).

4. Device according to Claim 3, characterised in that the first electrode (14) is stuck with one face on the printed circuit board (3) and has the free contact face (14′) on the side facing the insulating plate (16).

5. Device according to Claim 3, characterised in that the first electrode (14) is vaporised onto the pouring compound (20) as a film and has the free contact face (14′) on the side facing the insulating plate (16).

6. Device according to Claim 1, characterised in that the applied mains voltage of 220V (AC) or a direct-current voltage of 24V (DC) is transformed to a ten-fold or multiple value, for example to three kilovolts (kV), by the transformer (7) of the electric circuit member (1).

7. Device according to Claim 1, characterised in that the electric circuit member (1) is connected to a current source and has a chopper circuit (26) by means of which the necessary frequency for a converter (27) preceding the two electrodes (14, 15) is produced.

## Revendications

1. Appareil de stérilisation et de désodorisation d'espaces, constitué d'une partie de circuit électrique (1) muni d'un transformateur (7) pour produire une haute fréquence et une haute tension et une deuxième partie d'ionisation (2) munie d'une deuxième électrode (15) disposée en isolement électrique vis-à-vis d'une première électrode (14), à laquelle on applique la haute tension pour réaliser une décharge corona, caractérisé en ce qu'on dispose la partie de circuit électrique (1) sur une face du circuit imprimé (3) et directement sur son autre face la première électrode (14) en forme de plaque plane ainsi qu'une plaque isolante (16), on place la plaque isolante (16) et la première électrode (14) ainsi que le circuit imprimé (3) qui lui est relié en même temps que la partie de circuit (1) électrique qui lui est associé dans cet ordre ainsi qu'en montage par couches dans une deuxième électrode (15) en forme de capot et constituée d'une toile métallique ou une grille métallique mise à la terre.

2. Appareil selon la revendication 1, caractérisé en ce qu'on constitue la deuxième électrode (15) en forme de capot avec une surface de base (17) et des faces latérales (18) qui s'y raccordent, de sorte que la plaque isolante (16), la première électrode (14) ainsi que le circuit imprimé (3) avec la partie de circuit électrique (1) sont recouverts à l'état monté par les faces latérales (18), de telle sorte qu'on recouvre la partie de circuit électrique (1) sur la face opposée à la face de base (17) de la deuxième électrode (15) d'une masse moulée (20) sous forme d'une matière plastique appropriée comme du polychlorure de vinyle (PVC) ou du polyuréthanne (PU).

3. Appareil selon les revendications 1 et 2, caractérisé en ce que la partie de circuit électrique (1) avec le circuit imprimé (3) et la première électrode (14) sont incorporées dans une masse moulée, de sorte que la première électrode (14) présente une surface sans contact (14′) sur la face tournée vers la plaque isolante (16).

4. Appareil selon la revendication 3, caractérisé en ce que la première électrode (14) est collée par une force sur le circuit imprimé (3) et présente la surface sans contact (14′) sur la face dirigée vers la plaque isolante (16).

5. Appareil selon la revendication 3, caractérisé en ce que la première électrode (14) est vaporisée en tant que film sur la masse de moulage (20) et présente la surface exempte de contact (14′) sur la face tournée vers la plaque isolante (16).

6. Appareil selon la revendication 1, caractérisé en ce que le transformateur (7) de la partie de circuit électrique (1) transforme la tension alternative de réseau de 220 V ou une tension continue de 24 V en une valeur dix fois supérieure ou plus, par exemple à trois kilovolt (kV).

7. Appareil selon la revendication 1, caractérisé en ce que la partie de circuit électrique (1) est reliée à une source de courant et présente un hacheur (26) au moyen duquel on produit la fréquence nécessaire à un transformateur (27) branché en amont des deux électrodes (14, 15).
